# EUROPÄISCHE PATENTANMELDUNG

(11) **EP 0 839 519 A2**
(43) Veröffentlichungstag der Anmeldung: **06.05.1998**
(21) Anmeldenummer: 97250297.5
(22) Anmeldetag: 09.10.1997
(51) Int. Cl.: A61K 7/48

(54) **Protein- und vitaminhaltige Kosmetikzusammensetzung**

(30) Priorität: 09.10.1996 DE 19643365
(71) Anmelder: Wohner, Holger, 98000 Monaco (MC); Wohner, Christel, 98000 Monaco (MC)
(72) Erfinder: Wohner, Holger, 98000 Monaco (MC); Wohner, Christel, 98000 Monaco (MC)
(74) Vertreter: Walter, Wolf-Jürgen

(57) **Zusammenfassung**

Die Erfindung betrifft eine protein- und vitaminreiches Kosmetikzusammensetzung mit hohem Pflegewert aus natürlichen Wirkstoffen einschließlich Milch, vorzugsweise Schafsmilch. Erfindungsgemäß bereitgestellt wird eine protein- und vitaminhaltige Kosmetikzusammensetzung mit einem Wirkstoffgemisch, bestehend aus 5 - 90 Gew-% einer Säugermilch, die einen Proteigehalt von 1 bis 6 Gew-% hat, bezogen auf die wäßrige Originalform der Milch; 5 - 50 Gew-% β-Glucan aus Hefe; 0,5 - 5 Gew-% eines Algenextraktes; wobei der Anteil des Wirkstoffgemisches an der Gesamtmasse der Kosmetikzusammensetzung 0,5 bis 50 Gew-% betragen kann; und 50 - 99,5 Gew-% einer kosmetischen Basisformulierung, bezogen auf die Gesamtmasse der Kosmetikzusammensetzung.

## Beschreibung

Die Erfindung betrifft eine protein- und vitaminreiche Kosmetikzusammensetzung mit hohem Pflegewert.

Als Hautpflege wird die Stärkung und Unterstützung der natürlichen Hautfunktion verstanden, die insbesondere gegen Umwelteinwirkungwen durch allergische oder toxische Stoffe, Mikroorganismen, UV-Strahlung oder durch den Abgang körpereigener Elektrolyte, von Wasser und anderen Stoffen geschützt werden soll. Der Ausgleich, den die gesunde Haut normalerweise selbst vornimmt, reicht unter schlechten Bedingungen und bei empfindlicher Haut nicht aus und bedarf im allgemeinen einer Unterstützung durch zusätzliche Pflegestoffe. Dazu sind bereits eine Vielzahl von Präparaten entwickelt worden, deren Wirkungen, sehr unterschiedlich und im Einzelfall nicht immer ausreichend sind.

Milchprodukte sind in der DE-A-4411108 u.a. zur Herstellung von Hautpflegemitteln eingesetzt worden. Diese Milchprodukte sind jedoch einer mischfermentativen Behandlung mit Milchsäureprodukten und Hefen unterzogen worden.

In der DE-C-4344141 wird ein kosmetisches Produkt zur spezifischen Haarpflege beansprucht, das neben bestimmten Wirkstoffen auch Milchsäureliposomen enthält, wobei die Wirkungsrichtung bei einer besseren Haarregenerierung nach intensiven Umwelteinflüssen liegt.

Die DE-A-4408258 beschreibt eine Milch enthaltende Öl/Wasser-Emulsion, die polyethoxyliertes Vitamin E enthält, wodurch eine Stabilisierung der Milch gegen Oxidation, Hydrolyse und Ausfällung von Proteinen im sauren pH-Bereich ermöglicht wird.

Der Erfindung liegt die Aufgabe zugrunde, ein stabiles kosmetisches Produkt mit besonders hohen Pflegeeigenschaften aus natürlichen Wirkstoffen zu entwickeln, das einen Gehalt an Milch aufweist.

Erfindungsgemäß besteht die protein- und vitaminhaltige Kosmetikzusammensetzung aus einem Wirkstoffgemisch, bestehend aus
5 - 90 Gew-% einer Säugermilch, die einen Proteingehalt von 1 bis 6 Gew-% hat, bezogen auf die wäßrige Originalform der Milch;
5 - 50 Gew-% β-Glucan aus Hefe;
0,5 - 5 Gew-% eines Algenextraktes;
   wobei der Anteil des Wirkstoffgemisches an der Gesamtmasse der Kosmetikzusammensetzung 0,5 bis 50 Gew-% beträgt; und
50 - 99,5 Gew-% einer kosmetischen Basisformulierung, bezogen auf die Gesamtmasse der Kosmetikzusammensetzung.

Die Säugermilch ist vorzugsweise ausgewählt aus der Gruppe, die aus Schafsmilch, Eselsmilch, Stutenmilch, Ziegenmilch und Gemischen davon besteht. Sie kann auch in Form eines Trockenproduktes davon in entsprechenden Konzentrationen eingesetzt werden.

Der Gehalt an Säugermilch liegt vorzugsweise im Bereich von 25 bis 90 Gew-%, insbesondere 50 bis 90 Gew-%.

Der Gehalt an β-Glucan liegt vorzugsweise im Bereich von 15 bis 40 Gew-%, insbesondere 15 bis 30 Gew-%, wobei im allgemeinen 1,3 - 1,6-β-Glucan bevorzugt ist. Derartige β-Glucane sind Handelsprodukte, die im allgemeinen etwa 75 % reines β-Glucan enthalten.

Der Gehalt des Algenextraktes liegt vorzugsweise im Bereich von 0,8 bis 3 Gew-%. Als Algen werden eingesetzt Rotalgen, Braunalgen, Grünalgen und Blaualgen, wobei Rotalgen und Braunalgen bevorzugt sind.

Die Herstellung des Extraktes erfolgt generell nach bekannten Verfahren, wobei ein wäßriger Extrakt oder ein wasserlöslicher, getrockneter Propylenglycolextrakt verwendet wird. Handelsübliche Produkte, wie Extrakte von Phondros crispos (PHY-COLLCC®) können eingesetzt werden.

Unter dem Begriff "Säugermilch" im Sinne dieser Erfindung wird die entrahmte und nicht entrahmte flüssige Vollmilch von Säugern, wie Schafen, Eseln, Stuben und Ziegen verstanden sowie die entsprechenden Trockenprodukte dieser Milcharten. Auch die Milch anderer Säuger ist einsetzbar, wegen des geringeren Proteingehaltes jedoch nicht bevorzugt, da eine Ergänzung durch Proteinzusatz erforderlich wäre.

Bevorzugt ist eine Milch mit hohem Proteingehalt, z.B. mit einem Proteingehalt im Bereich von 30 bis 40 Gew-%, bezogen auf die Trockenmasse, oder von 3,5 bis 6 Gew-%, bezogen auf die wäßrige Originalform. Daher stellen Schafsmilch und Stutenmilch bevorzugte Bestandteile der erfindungsgemäßen Zusammensetzungen dar.

Die für Milch beschriebene Oxidation und Hydrolyse (DE-A-4408258) tritt bei der erfindungsgemäßen Zusammensetzung vermutlich durch das Zusammenwirken von β-Glucan, Algenextrakt und Milch nicht auf. Auch die schon im schwach sauren Bereich mögliche Proteinausfällung unterbleibt, so daß überraschenderweise ein lagerstabiles Produkt erhalten wird.

Die besondere Pflegewirkung ergibt sich aus der Kombination von hochproteinhalten Komponenten, dem Algenextrakt und dem β-Glucan, wodurch überraschenderweise selbst sehr empfindliche Haut, wie die von Kleinkindern, im hohen Maße auf natürliche Weise mit pflegenden und heilenden Wirkstoffen ausreichend versorgt wird und Rötungen, Hautrisse, Austrocknen und ähnliche Schädigungen vollständig unterbleiben. Dies gilt insbesondere auch für sensible Hautbereiche.

Die Wirkung wird noch verstärkt, wenn ein Teil des Wirkstoffgemisches verkapselt in Liposomen vorliegt neben einem unverkapselten Teil des Wirkstoffgemisches.

Ein besonders bevorzugtes Produkt ist daher eine kosmetische Zusammensetzung, bestehend aus 2 bis 20 Gew-% Liposomen mit den darin verkapselten Wirkstoffen Schafsmilch, 1.3-1.6-β-Glucan und Algenextrakt und daneben das gleiche Wirkstoffgemisch unverkapselt in einer Menge von 0,5 bis 40 Gew-%, bezogen auf die Gesamtmasse der kosmetischen Formulierung. Dabei liegt die Zusammensetzung des Wirkstoffgemisches in folgenden Bereichen Schafsmilch 20 bis 80 Gew-%, 1.3-1.6-β-Glucan 15 bis 30 Gew-%, Algenextrakt 0,8 bis 2,5 Gew-%.

Zur Herstellung der Liposomen, in die die Wirkstoffe verkapselt werden können, werden übliche Verfahren eingesetzt. Die Bilayerschicht der Liposomen kann durch Phospholipide unter Zusatz von Sterolen, wie Cholesterol, gebildet werden, wobei z.B. Dicetylphosphat als weiterer Zusatzstoff zweckmäßig ist, um ein Kollabieren der Vesikel zu verhindern. Beispielsweise können Liposomen aus einem Gemisch von Eilecithin, Dicetylphosphat und Cholesterol in einem molaren Verhältnis von 7:2:2 gebildet werden, wobei LUV- und MLV-Liposomen erhalten werden. Bevorzugt beträgt das Verhältnis 7:2:1 bis 7:2:3,5 (siehe z.B. Schieren et al., Biochim. et Biophys. Acta, Bd. 542, S. 137-153 (1978) oder Kinsky, Methods in Enzymology, XXXII Biomembranes, Teil B, S. 501-5123 (1974)). Es gibt auch eine Vielzahl weiterer Verfahren, die dem Fachmann auf diesem Gebiet bekannt sind.

Ebenso bekannt ist die Inkorporierung von wasserlöslichen Verbindungen bei niedriger Temperatur im Bereich von 10 bis 50 °C unter Rühren in die Vesikel.

Wäßrige Liposomsuspensionen können etwa 0,1 Vol-% bis etwa 50 Vol-% Liposomen enthalten, bezogen auf das Gesamtvolumen der Suspension.

Die erfindungsgemäße Kosmetikzusammensetzung kann neben den genannten Wirkstoffen in der Basisformulierung und neben Wasser zusätzlich Vitamine, wie Vitamin-B-Komplex, Vitamin E und Derivate davon oder andere radikalfangende Substanzen enthalten. Der Vitanmingehalt kann insgesamt bis zu 5 Gew-%, bezogen auf die Gesamtzuusammensetzung betragen. Weiterhin kann sie enthalten Carotinoide, Aminosäuren wie Glycin, Alanin, Valin, Leucin, Prolin, Serin usw. sowie andere dem Fachmann bekannte Stoffe, wie Cetearyl Alcohol, Glycerin, Ceteareth-20, Decyl Oleate, Glyceryl Stearate, Hydroxypropyl Guar, Octyl Dodecanol, Propylenglycol, Cyclomethicone, Acrylate C10-30 Alkylacrylate Crosspolymer, Allantoin, Jojobaöl usw.

Die Herstellung der erfindungsgemäßen Zusammensetzungen erfolgt vorteilhaft in der Weise, daß die Wirkstoffe Milch, β-Glucan und Algenextrakt, sowie gegebenenfalls weitere Wirkstoffe wie Vitamin-B-Komplex, Vitamin E und Enzyme, verrührt und anschließend sprühgetrocknet werden. Das erhaltene Granulat wird dann einer kosmetischen Basisformulierung, die auf übliche Weise hergestellt wird, in einem Anteil von 0,5 bis 50 Gew-% zugesetzt, bezogen auf das Gesamtgewicht.

Die besondere Pflegewirkung der erfindungsgemäßen Zusammensetzung kann durch das Vorhandensein von UV-Filtern wie 3-Benzylidencampher-Derivate, 4-Aminobenzoesäure-Derivate, Salicylsäureester, Benzophenon-Derivate, Salze der 2-Phenylbenzimidazol-5-sulfonsäure sowie Dibenzoylmethan-Derivate noch ergänzt werden. Ebenso können anorganische Filter, wie TiO₂ oder ZnO enthalten sein, die bevorzugt sind.

Die Erfindung soll nachstehend durch Beispiele näher erläutert werden.

### Beispiel 1

### 1A) Herstellung des Wirkstoffgemisches

74 g Schafsmilch wurden mit 25 g 1.3-1.6-β-Glucan bei einer Temperatur von nicht mehr als 15 °C verrührt, und es wurden 1 g eines Extraktes der Alge Phondos crispos unter weiterem Rühren bei etwa 500 U/Min bei dieser Temperatur hinzugegeben. Das Wirkstoffgemisch wurde bis zur Homogenität gerührt und anschließend einer Sprühtrocknung nach bekannten Verfahren unterworfen. Das erhaltene Granulat war das in den folgenden Beispielen eingesetzte Wirkstoffgemisch.

### 1B) Herstellung einer Creme

Es wurden verschiedene Phasen hergestellt. Die Phasen A und B wurden jeweils getrennt auf 65 ±5 °C erwärmt und miteinander unter Rühren vermischt. Danach erfolgte die Neutralisation mittels der Phase C. Bei einer Temperatur von 38 ±2 °C erfolgte dann die Zugabe von Phase D. Alle Angaben sind in Gewichtsprozent.

### Phase A

- Wasser: q.s.
- Glycerin: 2 %
- Acrylate C10-30 Alkylacrylate Crosspolymer: 0,5 %
- Propylenglycol: 2 %

### Phase B

- Cetearyl Alcohol: 3,0 %
- Octyl Stearate: 1,0 %

### Phase C

- Triethanolamin: 0,5 %

### Phase D

- Palmöl: 1,5 %
- Jojobaöl: 1,0 %
- Konservierungsmittel: 0,3 %
- Wirkstoff gemäß Beispiel 1A: 10,0 %
- Fragance: 0,3 %

### Beispiel 2

Es wurde wie in Beispiel 1 verfahren mit der Ausnahme, daß zusätzlich Liposomen mit einem Gehalt von 10 Gew-% Schafsmilch hergestellt wurden. Die Liposomensuspension wurde ebenfalls einer Sprühtrocknung unterworfen. Zusätzlich zu den 10 % Wirkstoff gemäß Beispiel 1A wurden 5 % des Liposomengranulates zugesetzt. Man erhielt eine Intensiv-Creme mit ausgezeichneter Pflegewirkung für empfindliche Hautbereiche.

### Beispiel 3

### Babypuder

- Talkum: q.s.
- Kaolin: 10 %
- Seidenprotein: 3,0 %
- Wirkstoff gemäß Beispiel 1A: 50 %

Die Bestandteile wurden in einem Mischer bei Umgebungstemperatur homogen miteinander vermischt.

### Beispiel 4

### Duschbad

Die nachfolgenden Rohstoffe wurden in das vorgegebene Wasser nacheinander unter Rühren gegeben, und anschließend wurde bis zum Erreichen der Homogenität weiter gerührt.
- Wasser: q.s.
- PEG-40 Hydrogenated Castor Oil: 3,0 %
- Sodium Laureth-2: 30 %
- Propylenglycol: 1,0 %
- Disodium Laureth: 20,0 %
- Glycerin: 1,0 %
- Rewoderm ES 90®: 2,0 %
- Wirkstoff gemäß Beispiel 1A: 1,5 %
- Fragance: 0,5

## Patentansprüche

1. Protein- und vitaminhaltige Kosmetikzusammensetzung, gekennzeichnet durch ein Wirkstoffgemisch, bestehend aus
5 - 90 Gew-% einer Säugermilch, die einen Proteingehalt von 1 bis 6 Gew-% hat, bezogen auf die wäßrige Originalform der Milch;
5 - 50 Gew-% β-Glucan aus Hefe;
0,5 - 5 Gew-% eines Algenextraktes;
wobei der Anteil des Wirkstoffgemisches an der Gesamtmasse der Kosmetikzusammensetzung 0,5 bis 50 Gew-% beträgt; und
50 - 99,5 Gew-% einer kosmetischen Basisformulierung, bezogen auf die Gesamtmasse der Kosmetikzusammensetzung.

2. Kosmetikzusammensetzung nach Anspruch 1, dadurch gekennzeichnet, daß die Säugermilch ausgewählt ist aus der Gruppe, die aus Schafsnilch, Eselsmilch, Stutenmilch, Ziegenmilch und Gemischen davon besteht, oder eine Trockenform davon ist.

3. Kosmetikzusammensetzung nach Anspruch 1, dadurch gekennzeichnet, daß der Gehalt an Säugermilch vorzugsweise im Bereich von 50 bis 90 Gew-% liegt.

4. Kosmetikzusammensetzung nach Anspruch 1, dadurch gekennzeichnet, daß der Gehalt an β-Glucan vorzugsweise im Bereich von 15 bis 40 Gew-%, insbesondere 15 bis 30 Gew-% liegt.

5. Kosmetikzusammensetzung nach Anspruch 1, dadurch gekennzeichnet, daß der Gehalt des Algenextraktes vorzugsweise im Bereich von 0,8 bis 3 Gew-% liegt.

6. Kosmetikzusammensetzung nach einem der Ansprüche 1 bis 5, dadurch gekennzeichnet, daß die Säugermilch Schafsmilch oder Stutenmilch ist, vorzugsweise Schafsmilch.

7. Kosmetikzusammensetzung nach einem der Ansprüche 1 bis 6, dadurch gekennzeichnet, daß das Wirkstoffgemisch wenigstens teilweise in Liposomen verkapselt vorliegt.

8. Kosmetikzusammensetzung nach Anspruch 7, dadurch gekennzeichnet, daß der Anteil des Wirkstoffgemisches, das in Liposomen verkapselt vorliegt, im Bereich von 2 bis 10 Gew-% liegt und der restliche Anteil unverkapselt ist.

9. Kosmetikzusammensetzung nach einem der Ansprüche 1 bis 7, dadurch gekennzeichnet, daß die Zusammensetzung zusätzlich kosmetische Wirkstoffe enthält, wie Vitamin-B-Komplex, Vitamin E, Aminosäuren, Aloe vera, Enzympräparate.
